# EUROPEAN PATENT APPLICATION

(11) **EP 4 119 143 A1**
(43) Date of publication of application: **18.01.2023**
(21) Application number: 21767375.5
(22) Date of filing: 11.03.2021
(51) Int. Cl.: A61K 31/519, A61K 31/53, A61K 31/4985, A61K 31/522, A61K 31/506, A61K 31/167, A61K 31/18, A61P 35/02

(54) **PHARMACEUTICAL COMPOSITION FOR PREVENTING OR TREATING NK/T-CELL LYMPHOMA OR NK-CELL LEUKEMIA, COMPRISING PHOSPHODIESTERASE TYPE 5 INHIBITOR**

(30) Priority: 11.03.2020 KR 20200030208
(71) Applicant: Samsung Life Public Welfare Foundation, Seoul 04348 (KR)
(72) Inventor: KIM, Won Seog, Seoul 06288 (KR); PARK, Chae Hwa, Seoul 06356 (KR); KIM, Joo Hyun, Seoul 06919 (KR)
(74) Representative: Cabinet Becker et Associés
(86) International application number: PCT/KR2021/003044
(87) International publication number: WO 2021/182897

(57) **Abstract**

The present invention relates to a pharmaceutical composition for preventing or treating NK/T-cell lymphoma, comprising a phosphodiesterase type 5 inhibitor, the pharmaceutical composition being for inhibiting the reactivation of the Epstein-Barr virus (EBV) induced by a HDAC inhibitor; an anticancer therapy adjuvant composition; and a health functional food. By using the pharmaceutical composition for preventing, alleviating or treating NK/T-cell lymphoma, according to the present invention, EBV reactivation, induced by a HDAC inhibitor used as an anticancer agent for NK/T-cell lymphoma, may be inhibited, and thus side-effects of the HDAC inhibitor may be reduced, and an anticancer effect may be notably increased. Thus, the present invention may be widely used in the pharmaceutical industry on the basis of said effects.

## Description

### [Technical Field]

The present invention relates to a pharmaceutical composition for preventing or treating natural killer (NK)/T-cell lymphoma or NK-cell leukemia, in which the pharmaceutical composition includes a phosphodiesterase type 5 inhibitor; an anticancer therapy adjuvant composition; and a health functional food. In particular, the present invention relates to a pharmaceutical composition for inhibiting reactivation of Epstein-Barr virus (EBV) induced by a histone deacetylase (HDAC) inhibitor.

### [Background Art]

Epstein-Barr virus (EBV) belongs to the herpesvirus family, and 90% or more of normal people become infected with EBV. EBV is usually established by a latent infection without symptoms, but in patients whose immune system is impaired over a long period of time due to a bone marrow or organ transplant, EBV is known to cause tumors such as lymphoproliferative diseases, Burkitt's lymphoma, nasopharyngeal carcinoma (NPC), Hodgkin's disease, etc. Lymphocyte diseases related to EBV include Hodgkin's lymphoma, NK/T-cell lymphoma other than nasal type lymph node, and post-transplant lymphoproliferative disease(PTLD). The lymphoproliferative disease, which is a non-Hodgkin's lymphoma type, is present with various clinical features ranging from benign diseases, such as polyclonal B-cell lymphocytosis, to malignant diseases, such as malignant lymphoma, as the proliferation of lymphocytes or plasma cells occurs as a result of EBV latency in the donor and the continued administration of immune inhibitors following solid organ or hematopoietic stem cell transplantation. The lymphoproliferative disease is a severe complication occurring throughout the whole body in 1-15% of organ transplant patients. It was recently confirmed that EBV genes are expressed in some patients with gastric cancer-in Japan and Korea, 5-10% of patients with gastric cancer. A research team at the University of Kagoshima analyzed the distribution of EBV-positive gastric cancer in seven countries, including Japan, and found that the odds ratios of EBV-associated gastric cancer were higher in Russia (1.9), Colombia (2.5), Chile (2.7), etc., compared to that in Japan (1.0).

Conventional treatments of EBV-associated tumors include reduction of immunosuppressants, use of antiviral drugs, chemotherapy, and administration of rituximab antibodies., and the like However, responses to such conventional treatments vary and an effective treatment for EBV-associated tumors has not been found yet. Thus, it is urgent to develop a new treatment for treating and curing EBV-associated tumors.

For adoptive immunotherapy using EBV antigen-specific T cells against EBV infection, Dr. Brenner's group conducted a study to find a method for treating EBV infections or EBV-associated tumors, such as EBV-associated lymphoma or throat cancer, by inducing CTLs specific for EBV antigens *in vitro* and transplanting hematopoietic stem cells. In Korea, the research group including the present inventors has succeeded initially in treating patients with EBV-positive lymphoma and acute leukemia using adoptive immunotherapy with antigen-specific NK/T cells. The adoptive immunotherapy using antigen-specific T cells cultured *in vitro* would help to overcome the limitations of an antibody drug. However, despite such an advantage of the adoptive immunotherapy, the costs for culturing antigen-specific T cells with high avidity *in vitro* which recognize and remove tumor cells by overcoming immune tolerance to a tumor-associated antigen are highly expensive, which is hindering its clinical applications for general purposes and commercialization.

Recently, there has been a lot of research on targeted immunotherapy using gene-engineered T cells as adoptive immunotherapy to overcome the limitations of antibody drugs, therapeutic cellular tumor vaccine, and adoptive cellular immunotherapy for treating tumor patients. In particular, it is expected that general-purpose targeted immunotherapy at the off-the-shelf stage which overcomes the limitations of tailor-made therapies may be developed by using TCR genes specific for tumor antigens. However, due to difficulties in obtaining tumor antigen-specific clones of T cells in order to obtain antigen-specific TCR genes and cloning the TCR genes, the development has only been modest in Korea and relevant clinical trials have been conducted to date by foreign researchers on a limited basis.

Histone protein is a protein that winds DNA strands, which are genetic information, in the cell nucleus, condenses DNA, and functions to increase or control gene expression through acetylation and methylation. When these acetylation and methylation decrease and fail to control, cancer may develop. Histone deacetylase (HDAC) functions to control the degree of histone acetylation and inhibit DNA expression. Abnormal overexpression of HDAC may cause cancer. As such, cancer may be induced by overexpression of HDAC. In order to inhibit this mechanism, an HDAC inhibitor is administered as an anticancer agent. However, the HDAC inhibitor in lymphoma patients induces reactivation of EBV, and there is no further treatment option after EBV reactivation occurs. Accordingly, currently, there is an urgent need to develop a therapeutic agent to prevent EBV reactivation in lymphoma.

### [Disclosure]

### [Technical Problem]

Accordingly, the present inventors identified that a phosphodiesterase type 5 inhibitor inhibited Epstein-Barr virus (EBV) reactivation induced by a histone deacetylase (HDAC) inhibitor while researching a substance capable of inhibiting the EBV reactivation induced by the HDAC inhibitor, and then completed the present invention.

Accordingly, an aspect of the present invention is to provide a pharmaceutical composition for preventing or treating natural killer (NK)/T-cell lymphoma or NK-cell leukemia, in which the pharmaceutical composition includes a phosphodiesterase type 5 inhibitor.

In addition, another aspect of the present invention is to provide a method for treating NK/T-cell lymphoma or NK-cell leukemia, in which the method includes administering a phosphodiesterase type 5 inhibitor to a subject.

Another aspect of the present invention is to provide an anticancer therapy adjuvant composition for preventing or treating NK-T cell lymphoma or NK-cell leukemia, in which the anticancer therapy adjuvant composition includes a phosphodiesterase type 5 inhibitor.

Another aspect of the present invention is to provide a health functional food for preventing or alleviating NK/T-cell lymphoma or NK-cell leukemia, in which the health functional food includes a phosphodiesterase type 5 inhibitor.

### [Technical Solution]

In order to achieve the above object, the present invention provides a pharmaceutical composition for preventing or treating natural killer (NK)/T-cell lymphoma or NK-cell leukemia, in which the pharmaceutical composition includes a phosphodiesterase type 5 inhibitor, and is for inhibiting reactivation of Epstein-Barr virus (EBV) by a histone deacetylase (HDAC) inhibitor.

In addition, the present invention provides a pharmaceutical composition for preventing or treating NK/T-cell lymphoma or NK-cell leukemia, in which the pharmaceutical composition includes a phosphodiesterase type 5 inhibitor and a HDAC inhibitor.

In order to achieve the above another object, the present invention provides a method for treating NK/T-cell lymphoma or NK-cell leukemia, in which the method includes administering a phosphodiesterase type 5 inhibitor to a subject, and is for inhibiting reactivation of EBV by an HDAC inhibitor.

In addition, the present invention provides a method for treating NK/T-cell lymphoma or NK-cell leukemia, in which the method includes administering a phosphodiesterase type 5 inhibitor and an HDAC inhibitor to a subject.

In order to achieve the above another object, the present invention provides an anticancer therapy adjuvant composition for preventing or treating NK-T cell lymphoma or NK-cell leukemia, in which the anticancer therapy adjuvant composition includes a phosphodiesterase type 5 inhibitor, and the NK/T-cell lymphoma or NK-cell leukemia is EBV-positive.

In order to achieve the above another object, the present invention provides a health functional food for preventing or alleviating NK/T-cell lymphoma or NK-cell leukemia, in which the health functional food includes a phosphodiesterase type 5 inhibitor, and the NK/T-cell lymphoma or NK-cell leukemia is EBV-positive.

In addition, The present invention provides a health functional food for preventing or alleviating NK/T-cell lymphoma or NK-cell leukemia, in which the health functional food includes a phosphodiesterase type 5 inhibitor and an HDAC inhibitor, and the NK/T-cell lymphoma or NK-cell leukemia is EBV-positive.

### [Advantageous Effects]

When the phosphodiesterase type 5 inhibitor according to the present invention is used, Epstein-Barr virus reactivation, induced by a histone deacetylase (HDAC) inhibitor used as an anticancer agent for natural killer (NK)/T-cell lymphoma or NK-cell leukemia, may be inhibited, and thus side-effects of the HDAC inhibitor may be reduced, and an anticancer effect may be notably increased. Thus, the present invention may be widely used in the pharmaceutical industry on the basis of said effects.

### [Description of Drawings]

FIG. 1A is a diagram identifying the expression of BZLF1 by varying the treatment time when SNK6 cells are treated with romidepsin and sildenafil, respectively, alone or simultaneously.
FIG. 1B is a diagram identifying the expression of BZLF1 by varying the treatment time when NK92MI cells are treated with romidepsin and sildenafil, respectively, alone or simultaneously.
FIG. 2A is a diagram identifying the expression of BZLF1 by varying the treatment time of sildenafil when SNK6 cells are treated with romidepsin and sildenafil, respectively, alone or simultaneously.
FIG. 2B is a diagram identifying the expression of BZLF1 by varying the treatment time of sildenafil when NK92MI cells are treated with romidepsin and sildenafil, respectively, alone or simultaneously.
FIG. 3A is a diagram identifying through mRNA expression of BZLF1 whether EBV reactivation induced by romidepsin is shown in an EBV-positive cell line (Daudi) other than NK/T-cell lymphoma or NK-cell leukemia cell lines.
FIG. 3B is a diagram identifying through mRNA expression of BRLF1 whether EBV reactivation induced by romidepsin is shown in an EBV-positive cell line (Daudi) other than NK/T-cell lymphoma or NK-cell leukemia cell lines.
FIG. 3C is a diagram identifying through mRNA expression of BZLF1 whether EBV reactivation induced by romidepsin is shown in an EBV-positive cell line (Raji) other than NK/T-cell lymphoma or NK-cell leukemia cell lines.
FIG. 3D is a diagram identifying through mRNA expression of BRLF1 whether EBV reactivation induced by romidepsin is shown in an EBV-positive cell line (Raji) other than NK/T-cell lymphoma or NK-cell leukemia cell lines.
FIG. 3E is a diagram identifying through mRNA expression of BZLF1 whether EBV reactivation induced by romidepsin is shown in an EBV-positive cell line (1A2) other than NK/T-cell lymphoma or NK-cell leukemia cell lines.
FIG. 3F is a diagram identifying through mRNA expression of BRLF1 whether EBV reactivation induced by romidepsin is shown in an EBV-positive cell line (1A2) other than NK/T-cell lymphoma or NK-cell leukemia cell lines.
FIG. 4A is a diagram identifying through EBV copy number whether sildenafil has the effect of inhibiting EBV reactivation induced by romidepsin in an EBV-positive cell line (Daudi) other than NK/T-cell lymphoma or NK-cell leukemia cell lines.
FIG. 4B is a diagram identifying through mRNA expression of BZLF1 whether sildenafil has the effect of inhibiting EBV reactivation induced by romidepsin in an EBV-positive cell line (Daudi) other than NK/T-cell lymphoma or NK-cell leukemia cell lines.
FIG. 4C is a diagram identifying through mRNA expression of BRLF1 whether sildenafil has the effect of inhibiting EBV reactivation induced by romidepsin in an EBV-positive cell line (Daudi) other than NK/T-cell lymphoma or NK-cell leukemia cell lines.
FIG. 5A is a diagram identifying through EBV copy number whether sildenafil inhibits EBV reactivation induced by an HDAC inhibitor in SNK6 cells.
FIG. 5B is a diagram identifying through mRNA expression of BZLF1 whether sildenafil inhibits EBV reactivation induced by an HDAC inhibitor in SNK6 cells.
FIG. 5C is a diagram identifying through mRNA expression of BRLF1 whether sildenafil inhibits EBV reactivation induced by an HDAC inhibitor in SNK6 cells.
FIG. 5D is a diagram identifying that the survival rate of tumor cells is reduced by the inhibition of EBV reactivation induced by sildenafil in SNK6 cells.
FIG. 6A is a diagram identifying through mRNA expression of BRLF5 whether sildenafil inhibits EBV reactivation induced by an HDAC inhibitor in NK92MI cells.
FIG. 6B is a diagram identifying through mRNA expression of BZLF1 whether sildenafil inhibits EBV reactivation induced by an HDAC inhibitor in NK92MI cells.
FIG. 6C is a diagram identifying through mRNA expression of BRLF1 whether sildenafil inhibits EBV reactivation induced by an HDAC inhibitor in NK92MI cells.
FIG. 6D is a diagram identifying that the survival rate of tumor cells is reduced by the inhibition of EBV reactivation induced by sildenafil in SNK6 cells.
FIG. 7A is a diagram identifying through mRNA expression of BZLF1 whether the effect of inhibiting EBV reactivation induced by romidepsin is shown by the PDE5 inhibitor when SNK6 cells are treated with vardenafil, which is a PDE5 inhibitor, alone or treated with the romidepsin simultaneously.
FIG. 7B is a diagram identifying through mRNA expression of BRLF1 whether the effect of inhibiting EBV reactivation induced by romidepsin is shown by the PDE5 inhibitor when SNK6 cells are treated with vardenafil, which is a PDE5 inhibitor, alone or treated with the romidepsin simultaneously.
FIG. 7C is a diagram identifying through mRNA expression of BZLF1 whether the effect of inhibiting EBV reactivation induced by romidepsin is shown by the PDE5 inhibitor when SNK6 cells are treated with udenafil, which is a PDE5 inhibitor, alone or treated with the romidepsin simultaneously.
FIG. 7D is a diagram identifying through mRNA expression of BRLF1 whether the effect of inhibiting EBV reactivation induced by romidepsin is shown by the PDE5 inhibitor when SNK6 cells are treated with udenafil, which is a PDE5 inhibitor, alone or treated with the romidepsin simultaneously.
FIG. 7E is a diagram identifying through mRNA expression of BZLF1 whether the effect of inhibiting EBV reactivation induced by romidepsin is shown by the PDE5 inhibitor when SNK6 cells are treated with tadalafil, which is a PDE5 inhibitor, alone or treated with the romidepsin simultaneously.
FIG. 7F is a diagram identifying through mRNA expression of BRLF1 whether the effect of inhibiting EBV reactivation induced by romidepsin is shown by the PDE5 inhibitor when SNK6 cells are treated with tadalafil, which is a PDE5 inhibitor, alone or treated with the romidepsin simultaneously.

### [Modes of the Invention]

Hereinafter, exemplary embodiments of the present invention will be described in detail.

The present invention provides a pharmaceutical composition for preventing or treating natural killer (NK)/T-cell lymphoma or NK-cell leukemia, in which the pharmaceutical composition includes a phosphodiesterase type 5 inhibitor, and is for inhibiting the reactivation of Epstein-Barr virus (EBV) induced by a histone deacetylase (HDAC) inhibitor.

In an embodiment of the present invention, the NK/T-cell lymphoma or NK-cell leukemia may be EBV-positive NK/T-cell lymphoma or NK-cell leukemia, preferably EBV-positive NK/T-cell lymphoma or NK-cell leukemia in which EBV is reactivated by an HDAC inhibitor.

In the present invention, the phosphodiesterase 5 inhibitor (PDE5 inhibitor) is known as a drug that inhibits a degrading action of cGMP-specific phosphodiesterase type 5 in vascular smooth muscle cells that supply blood to the corpus cavemosum of the penis. Accordingly, it is used to treat erectile dysfunction, and PDE5 is also known to be effective in treating pulmonary arterial hypertension because it is also present in an arterial vessel wall. However, it is not known about the effect that may inhibit the side effects of an HDAC inhibitor as in the present invention.

In an embodiment of the present invention, the phosphodiesterase type 5 inhibitor may be one or more selected from the group consisting of sildenafil, mirodenafil, vardenafil, tadalafil, udenafil, dasantafil, and avanafil, and preferably sildenafil, vardenafil, tadalafil or udenafil, but is not limited thereto.

In an embodiment of the present invention, the HDAC inhibitor may have an anticancer effect. However, when the HDAC inhibitor is administered to a patient with NK/T-cell lymphoma or NK-cell leukemia, reactivation of EBV may be induced to inhibit the anticancer effect. The phosphodiesterase type 5 inhibitor of an embodiment of the present invention may inhibit reactivation of EBV induced by an HDAC inhibitor.

In an embodiment of the present invention, the HDAC inhibitor may be one or more selected from the group consisting of romidepsin, vorinostat, panobinostat, belinostat, entinostat, mocetinostat, givinostat, pracinostat, quisinostat, abexinostat, chr-3996, and AR-42.

In the present invention, the pharmaceutical composition may be used for inhibiting the reactivation of the EBV induced by an HDAC inhibitor.

The term "prevention" as used herein means all actions that inhibit reactivation of the EBV or delay the progression thereof through the administration of the composition of an embodiment of the present invention.

The term "treatment" as used herein means all actions that alleviate or beneficially change diseases caused by the virus, such as Lymphoproliferative disease, Burkitt's lymphoma, nasopharyngeal carcinoma (NPC), and Hodgkin's disease, by inhibiting the reactivation of the EBV by administration of the composition of an embodiment of the present invention, and means an attempt to obtain beneficial or desired results, including clinical results. Beneficial or desired clinical results may include, but are not limited to, alleviation or amelioration of one or more symptoms or conditions; diminishment of extent of disease; stabilization of the state of disease; prevention of development of disease; prevention of spread of disease; delay or slowing of disease progression, delay or slowing of disease onset, amelioration or palliation of the disease state; and remission (whether partial or total), whether detectable or undetectable. The term "treatment" may also mean prolonging survival of a patient beyond that expected in the absence of treatment. In addition, the term "treatment" may mean inhibiting the progression of disease or slowing the progression of disease temporarily, although more preferably, it involves halting the progression of the disease permanently. In an embodiment of the present invention, the term "treatment" may mean improving the survival of a patient preferably by inhibiting the side effects of the HDAC inhibitor to enhance the anticancer effect of the HDAC inhibitor.

The pharmaceutical composition of an embodiment of the present invention may be formulated in the form of oral preparations such as powder, granule, tablet, capsule, suspension, emulsion, syrup or aerosol, external preparation, suppositories, or sterilized injection solutions according to each conventional method. The carriers, excipients, and diluents that may be included in the pharmaceutical composition are exemplified by lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. Formulations may be prepared by using generally used excipients or diluents such as fillers, extenders, binders, wetting agents, disintegrating agents, and surfactants. Solid formulations for oral administration include tablets, pills, powders, granules, and capsules. These solid formulations are prepared by mixing the extract or compound of an embodiment of the present invention with one or more excipients such as starch, calcium carbonate, sucrose or lactose, gelatin, etc. Except for the simple excipients, lubricants, for example, magnesium stearate, and talc may be used. Liquid formulations for oral administrations include suspensions, solutions, emulsions, and syrups, and the above-mentioned formulations may include various excipients such as wetting agents, sweeteners, aromatics, and preservatives in addition to generally used simple diluents such as water and liquid paraffin. Formulations for parenteral administration include sterilized aqueous solutions, water-insoluble excipients, suspensions, emulsions, lyophilized preparations, and suppositories. Water insoluble excipients and suspensions may include propylene glycol, polyethylene glycol, vegetable oil like olive oil, injectable ester like ethylolate, etc. Suppositories may include witepsol, macrogol, tween 61, cacao butter, laurin butter, glycerogelatin, etc.

A dose of the pharmaceutical composition of an embodiment of the present invention will vary depending on the age, gender, and body weight of a subject to be treated, particular disease or pathological condition to be treated, severity of the disease or pathological condition, administration route, and decision by a physician. The determination of the dose based on such factors is within the knowledge of those skilled in the art. A general dose is 0.01 mg/kg/day to about 2,000 mg/kg/day, and more preferably 0.1 mg/kg/day to 1,000 mg/kg/day. The administration may be carried out once a day or divided several times. The dose is not intended to limit the scope of an embodiment of the present invention in any way.

The pharmaceutical composition of an embodiment of the present invention may be administered to mammals such as mice, livestock, humans, etc. through various routes. All routes of administration may be expected, for example, the pharmaceutical composition may be administered orally, intrarectally, intravenously, intramuscularly, subcutaneously, intrauterinely, intradurally, or intracerebrovascularly.

In an embodiment of the present invention, the pharmaceutical composition for preventing or treating NK/T-cell lymphoma or NK-cell leukemia may further include any compound or natural extract that is known to have anticancer activity and has presently been verified for safety to increase and reinforce an anticancer effect, in addition to active ingredients.

When the pharmaceutical composition of an embodiment of the present invention is used, the reactivation of EBV by the HDAC inhibitor is inhibited, and NK/T-cell lymphoma or NK-cell leukemia can be effectively prevented or treated.

Accordingly, the present invention provides a method for treating NK/T-cell lymphoma or NK-cell leukemia, in which the method includes administering a phosphodiesterase type 5 inhibitor to a subject, and is for inhibiting the reactivation of EBV by an HDAC inhibitor.

As used herein, the term "subject" refers to a subject having a cancer disease, preferably a mammal such as a horse, a sheep, a pig, a goat, and a dog, including a human, who has a cancer disease for which an HDAC inhibitor has a therapeutic effect and may show an enhanced therapeutic effect by inhibiting EBV reactivation induced by the HDAC inhibitor when the phosphodiesterase type 5 inhibitor is administered according to an embodiment of the present invention, preferably a human.

In addition, the present invention provides a pharmaceutical composition for preventing or treating NK/T-cell lymphoma or NK-cell leukemia, in which the pharmaceutical composition includes a phosphodiesterase type 5 inhibitor and an HDAC inhibitor.

When the pharmaceutical composition of an embodiment of the present invention is used, the reactivation of EBV is inhibited, and the anticancer effect produced by the HDAC inhibitor works effectively, so that NK/T-cell lymphoma or NK-cell leukemia can be effectively prevented or treated.

In addition, the pharmaceutical composition of an embodiment of the present invention may be used alone or in combination with surgery, radiation treatment, hormone treatment, chemotherapy, a method of using a biological response modifier, or the like, to prevent or treat NK/T-cell lymphoma or NK-cell leukemia.

In addition, the present invention provides a method for treating NK/T-cell lymphoma or NK-cell leukemia, in which the method includes administering a phosphodiesterase type 5 inhibitor and an HDAC inhibitor to a subject.

In the method for treating NK/T-cell lymphoma or NK-cell leukemia, the term "subject" is as described above.

In addition, the present invention provides an anticancer therapy adjuvant composition for preventing or treating NK-T cell lymphoma or NK-cell leukemia, in which the anticancer therapy adjuvant composition includes a phosphodiesterase type 5 inhibitor, and the NK/T-cell lymphoma or NK-cell leukemia is EBV-positive.

The EBV-positive NK/T-cell lymphoma or NK-cell leukemia may be one in which EBV is reactivated by an HDAC inhibitor, and the phosphodiesterase type 5 inhibitor may enhance the anticancer therapeutic effect of the HDAC inhibitor by inhibiting the reactivation of EBV.

As used herein, the term "anticancer therapy adjuvant composition" means a composition that synergistically increases the effect of anticancer treatment by preventing side effects caused by an anticancer agent when applied in parallel with treatment with the anticancer agent.

Accordingly, the anticancer therapy adjuvant composition may be used as an adjuvant for cancer treatment using an HDAC inhibitor or other anticancer agents, and may be administered simultaneously or sequentially with an anticancer agent as an anticancer adjuvant.

In addition, the present invention provides a health functional food for preventing or alleviating NK/T-cell lymphoma or NK-cell leukemia, in which the health functional food includes a phosphodiesterase type 5 inhibitor, and the NK/T-cell lymphoma or NK-cell leukemia is EBV-positive.

In addition, the present invention provides a health functional food for preventing or alleviating NK/T-cell lymphoma or NK-cell leukemia, in which the health functional food includes a phosphodiesterase type 5 inhibitor and an HDAC inhibitor, and the NK/T-cell lymphoma or NK-cell leukemia is EBV-positive.

The EBV-positive NK/T-cell lymphoma or NK-cell leukemia may be one in which EBV is reactivated by an HDAC inhibitor, and the phosphodiesterase type 5 inhibitor may inhibit the reactivation of the EBV to prevent the onset of diseases caused by the virus, such as Lymphoproliferative disease, Burkitt's lymphoma, nasopharyngeal carcinoma (NPC), and Hodgkin's disease, or to improve conditions.

The health functional food according to an embodiment of the present invention includes all types of food including functional food, nutritional supplement, health food, and food additives. These types of health functional food may be prepared in various forms by conventional methods known in the art.

For example, as a health food, the health functional food of an embodiment of the present invention may be ingested by granulating, encapsulating and powdering itself, or may be prepared in the form of tea, juice, and drink for drinking purpose. In addition, the health functional food of an embodiment of the present invention may be prepared in the form of a composition by mixing the same with a known substance or active ingredient known to have an alleviating or therapeutic effect on NK/T-cell lymphoma or NK-cell leukemia. In particular, the known substance may be a substance that causes the EBV to be reactivated, but is not limited thereto.

In addition, the functional foods may be prepared by adding the health functional food of an embodiment of the present invention to beverages (including alcoholic beverages), fruits and processed foods thereof (for example, canned fruits, bottled jam, and marmalade), fish, meat and processed foods thereof (for example, ham and corn beef sausage), bread and noodles (for example, udon, buckwheat noodles, ramen noodles, spaghetti, and macaroni), juice, various drinks, cookies, taffy, dairy product (for example, butter and cheese), edible vegetable oil, margarine, vegetable protein, retort food, frozen food, and various kinds of seasoning (for example, soybean paste, soy sauce, and sauce).

The preferred content of the health functional food of an embodiment of the present invention is not limited thereto, but may be, for example, 0.01 to 80% by weight of the finally manufactured food, and preferably 0.01 to 50% by weight of the finally manufactured food.

In addition, in order to use the health functional food of an embodiment of the present invention in the form of a food additive, it may be prepared and used in the form of a powder or a concentrate.

The overlapping content is omitted in consideration of complexity of the present specification. Terms not otherwise defined herein have meanings commonly used in the technical field to which the present invention belongs.

Hereinafter, the present invention will be described in detail based on examples. However, following examples are merely illustrative of the present invention and thus, contents of the present invention are not limited to the following examples.

### Example 1. Identification of EBV Reactivation Inhibitory Effect by HDAC Inhibitor of Sildenafil

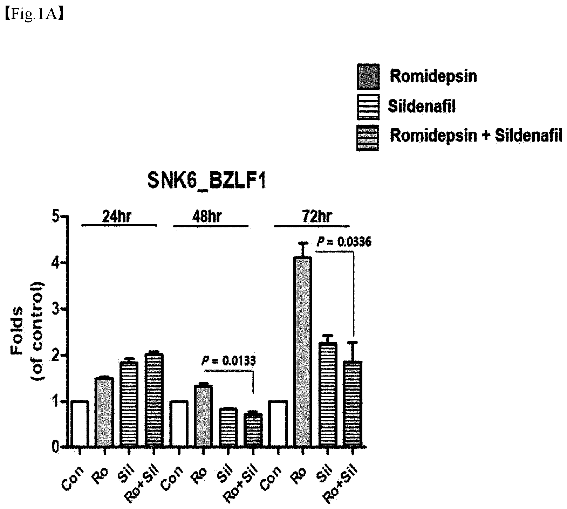

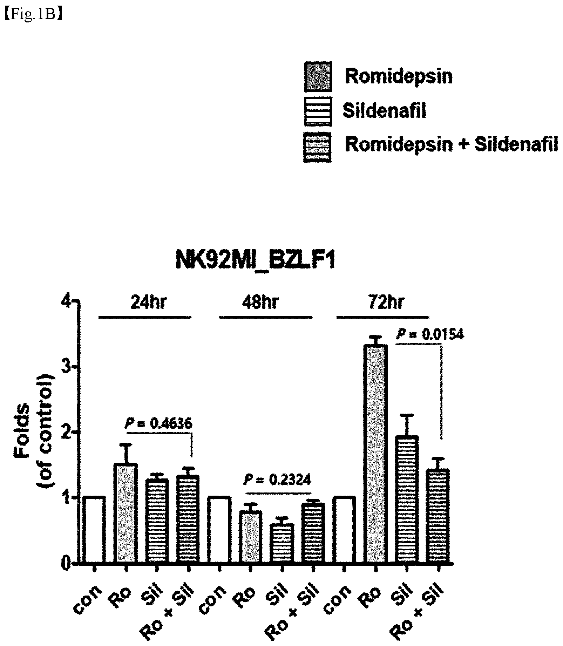

The NK/T-cell lymphoma cell line SNK6 (source; Seoul National University) and the NK-cell leukemia cell line NK92MI (source; Seoul National University) were treated with the HDAC inhibitor, romidepsin 10 nM, and the PDE5 inhibitor, sildenafil 100 nM, for 24, 48, and 72 hours. After treatment, cells were harvested every hour. Then, RNA was extracted using TRIzol reagent (Ambion, Life technologies), cDNA synthesis was performed using Omniscript RT kit (Qiagen), and the expression of BZLF1, an mRNA expressed during EBV reactivation, was identified through real-time PCR. The group treated with DMSO only was used as a control group. The results for SNK6 cells are shown in FIG. 1A, the results for NK92MI cells are shown in FIG. 1B. The primer sequences used in real-time PCR are shown in Table 1.

**[Table 1]**

| **Primer names** | **Sequences (5' -> 3')** | **SEQ ID NOS.** |
|---|---|---|
| BZLF1-Forward primer | AAT GCC GGG CCA AGT TTA AGC AAC | 1 |
| BZLF1-reverse primer | TTG GGC ACA TCT GCT TCA ACA GGA | 2 |
| BRLF1-Forward primer | TGG CTT GGA AGA CTT TCT GAG GCT | 3 |
| BRLF1-reverse primer | AAT CTC CAC ACT CCC GGC TGT AAA | 4 |
| GAPDH-Forward primer | GGC ATG GAC TGT GGT CAT GAG | 5 |
| GAPDH-reverse primer | TGC ACC ACC AAC TGC TTA GC | 6 |

As shown in FIGS. 1A and 1B, when romidepsin alone was treated, it was identified that the expression of BZLF1 increased in SNK6 cells and NK92MI cells compared to the control group, identifying that EBV was reactivated. On the other hand, when romidepsin and sildenafil were treated together for 72 hours, it was identified that the expression of BZLF1 was significantly decreased compared to the group treated with romidepsin alone, identifying that sildenafil inhibited the reactivation of EBV increased by romidepsin.

### Example 2. Identification of EBV Reactivation Inhibitory Effect by HDAC Inhibitors by Sildenafil Concentration

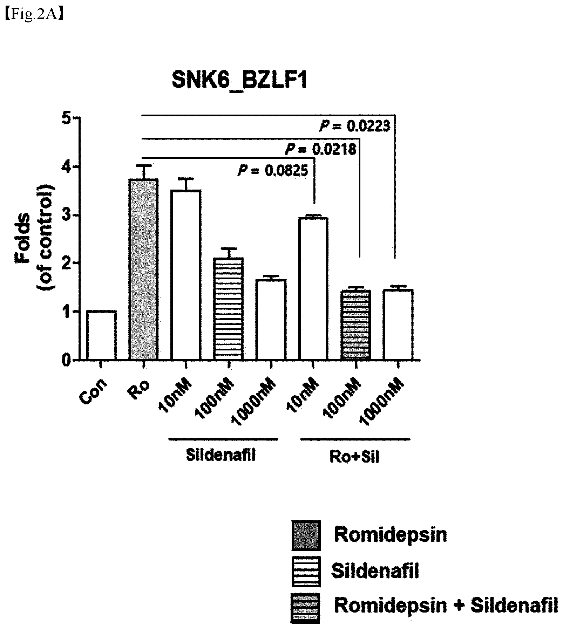

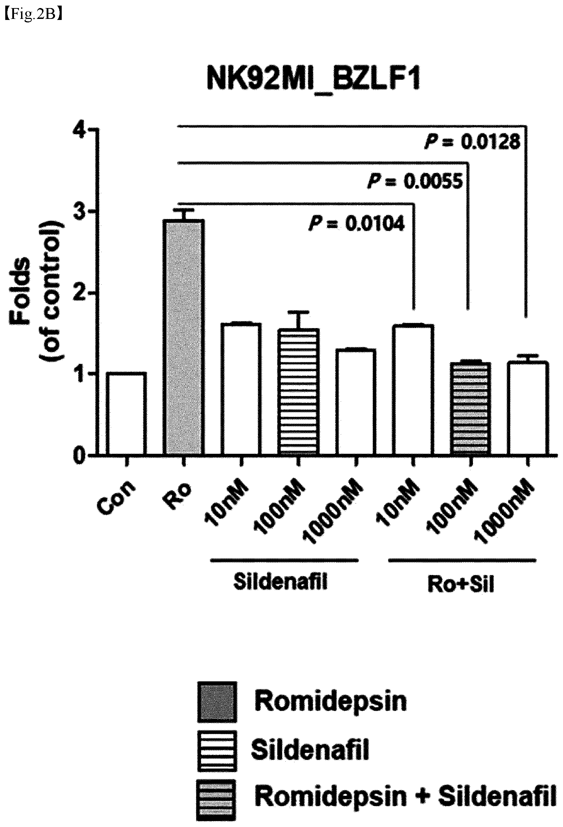

BZLF1 mRNA expression was measured in the same manner as in Example 1, with a group treated with sildenafil alone and a group treated with 10 nM of romidepsin simultaneously at a concentration of sildenafil of 10, 100 or 1000 nM in SNK6 and NK92MI cells as experimental groups. The treatment time was 72 hours. The results for SNK cells are shown in FIG. 2A, and the results for NK92MI cells are shown in FIG. 2B.

As shown in FIGS. 2A and 2B, it was identified that, when sildenafil was treated in combination, the effect of inhibiting the reactivation of EBV by romidepsin was observed.

### Example 3. Verification of NK/T Cell Lymphoma or NK-cell leukemia Specificity of EBV Reactivation Inhibitory Effect by HDAC Inhibitor of Sildenafil

### 3.1 Selection of Non-NK/T Cell Lymphoma and Non-NK-cell leukemia Cell Lines with EBV Reactivation by Romidepsin

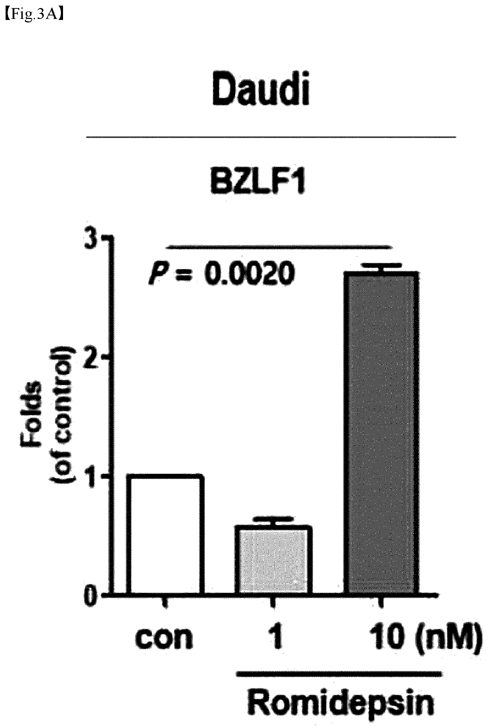

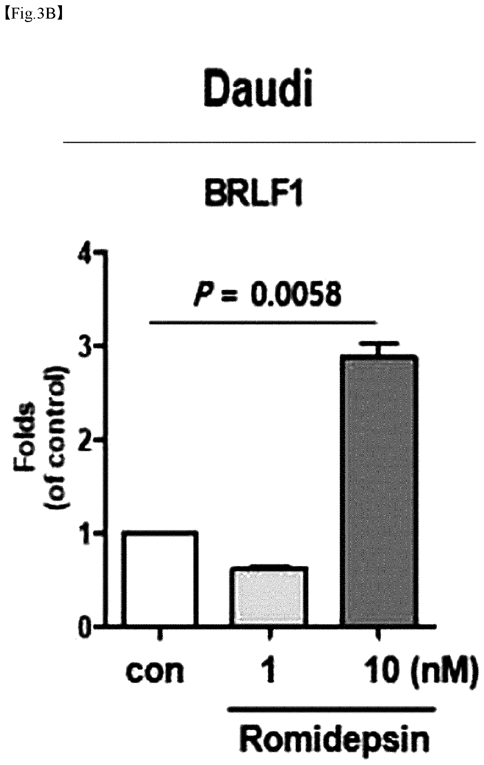

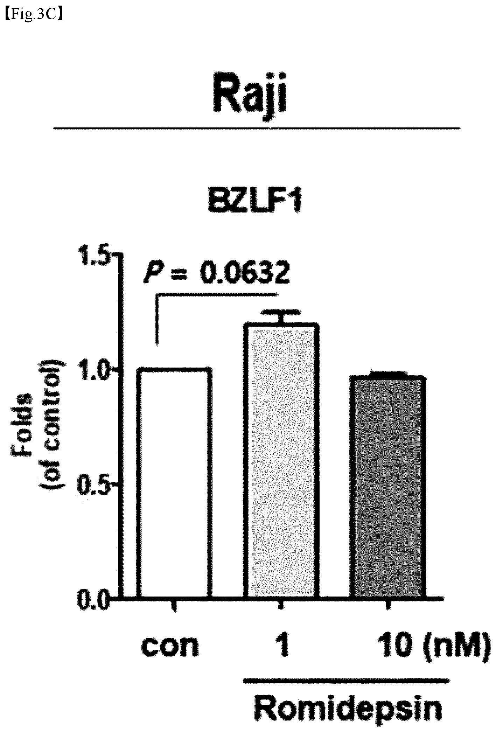

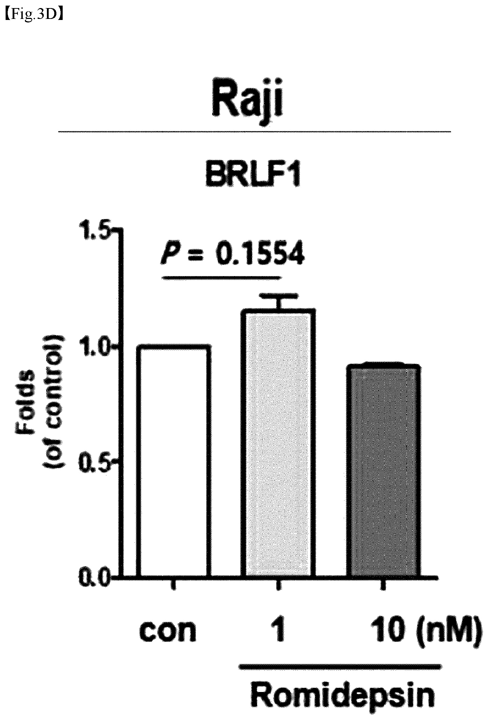

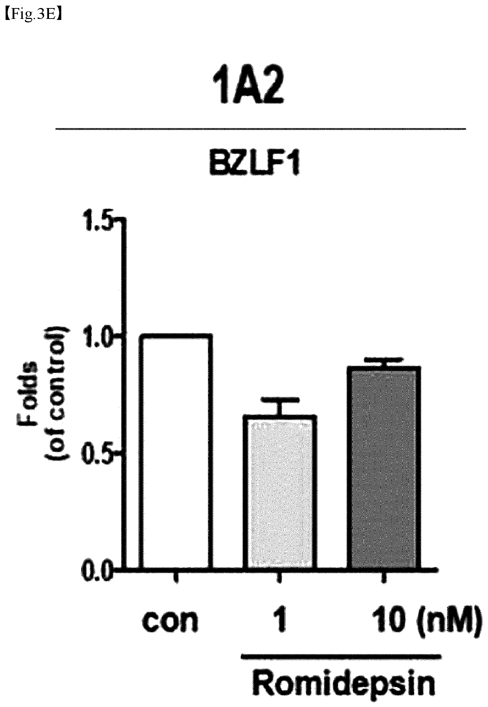

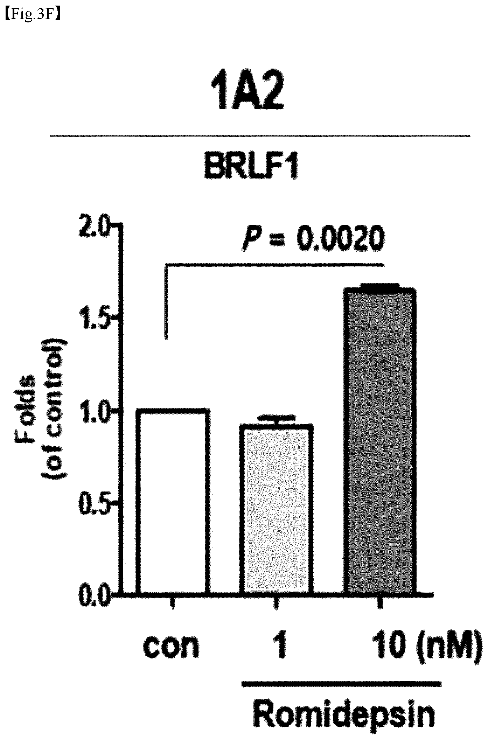

EBV-positive cell lines other than SNK6 and NK92MI were collected to identify EBV reactivity by romidepsin and the effect of sildenafil. As EBV-positive cell lines, the Daudi cell line (ATCC, CCL-213) and Raji cell line (ATCC, CCL-86), which are Burkitt's lymphoma cells, and the 1A2 cell line (ATCC, CRL-8119), which is a lymphoma cell, were treated with 1 or 10 nM of romidepsin for 72 hours, and mRNA expression of BZLF1 and BRLF1, genes whose expression is increased in EBV reactivation, was identified. The results for BZLF1 mRNA expression of the Daudi cell line are shown in FIG. 3A, and the results for BRLF1 mRNA expression are shown in FIG. 3B. In addition, the results for BZLF1 mRNA expression of the Raji cell line are shown in FIG. 3C, and the results for BRLF1 mRNA expression are shown in FIG. 3D. In addition, the results for BZLF1 mRNA expression of the 1A2 cell line are shown in FIG. 3E, and the results for BRLF1 mRNA expression are shown in FIG. 3F.

As shown in FIGS. 3A to 3F, it was identified that the expressions of BZLF1 and BRLF1 were increased by romidepsin treatment in the Daudi cell line. In the case of the Raji cell line, it was identified that the expressions of BZLF1 and BRLF1 were not increased, and in the case of the 1A2 cell line, it was identified that only the expression of BRLF1 was increased. Accordingly, it was identified that EBV reactivation by romidepsin appeared only in the Daudi cell line.

### 3.2 Verification of NK/T Cell Lymphoma or NK-cell leukemia Cell Specificity of EBV Reactivation Inhibitory Effect by HDAC Inhibitor of Sildenafil

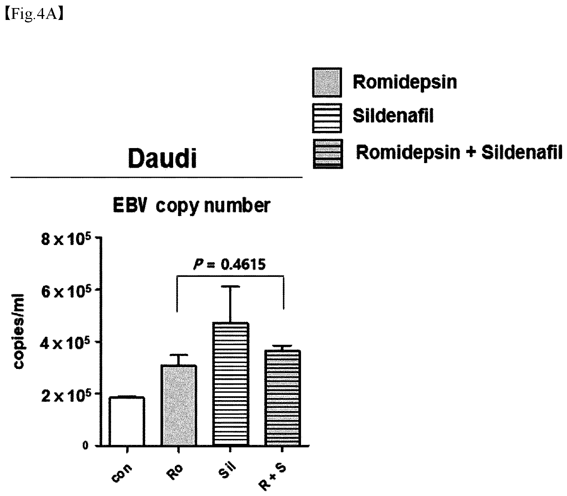

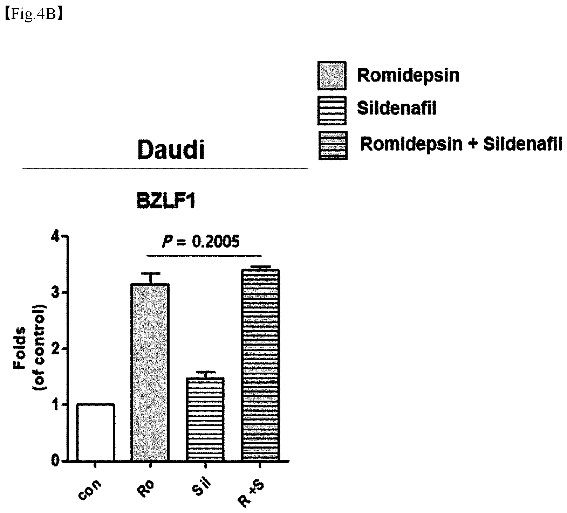

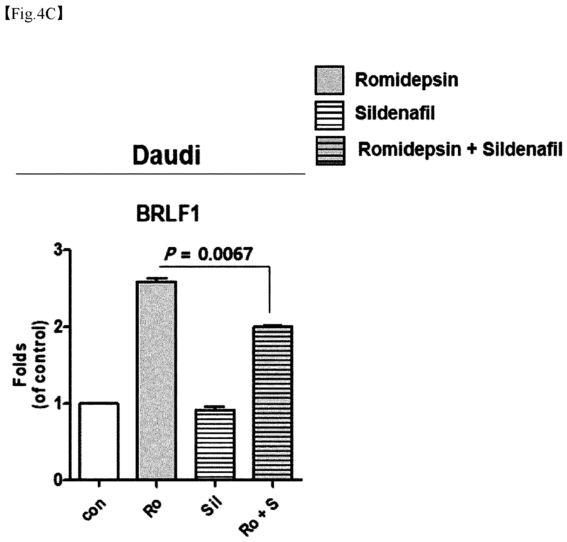

When sildenafil and romidepsin were simultaneously treated in the Daudi cell line, which is a cell line showing EBV reactivation by romidepsin identified in Example 3.1, an experiment was performed to determine whether EBV reactivation by romidepsin was inhibited. In the Daudi cell line, a group treated with 10 nM of romidepsin and 100 nM of sildenafil separately for 72 hours and a group treated therewith simultaneously were used as experimental groups, and a group treated with DMSO only was used as a control group. In order to measure the EBV copy number secreted out of the cell by EBV reactivation, by extracting DNA from the medium and measuring the Ct value by real-time PCR and comparing the same with the reference EBV number, the EBV copy number was measured and shown in FIG. 4A. In the same manner as in Example 3.1, BZLF1 mRNA expression was measured and shown in FIG. 4B, and BRLF1 mRNA was measured and shown in FIG. 4C.

As shown in FIGS. 4A to 4C, no significant difference was observed between the group treated with romidepsin alone and the group treated with romidepsin and sildenafil simultaneously in the Daudi cell line. Accordingly, it was identified that, in the case of non-NK/T-cell lymphoma or non-NK-cell leukemia cells, the inhibitory effect on EBV reactivation by sildenafil did not appear.

### Example 4. Identification of EBV Inhibitory Effect by Sildenafil

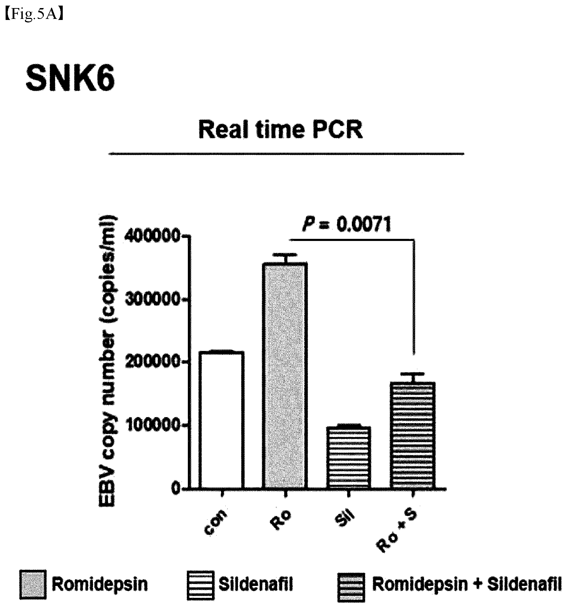

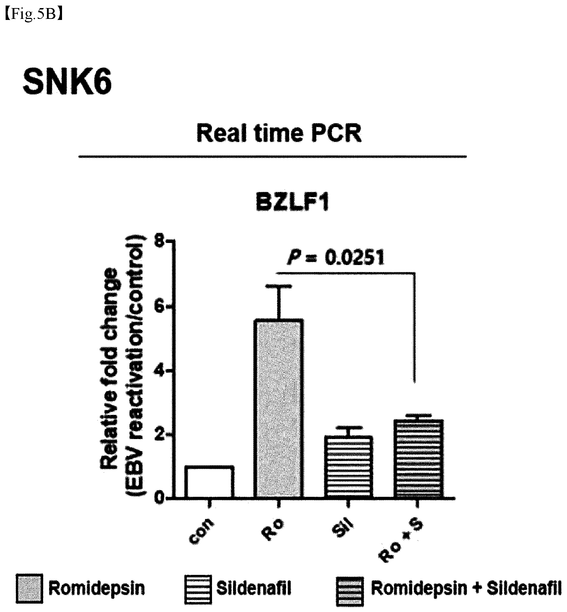

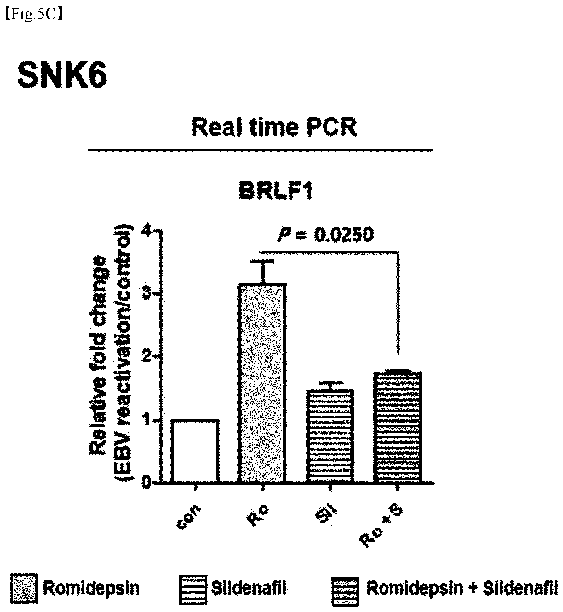

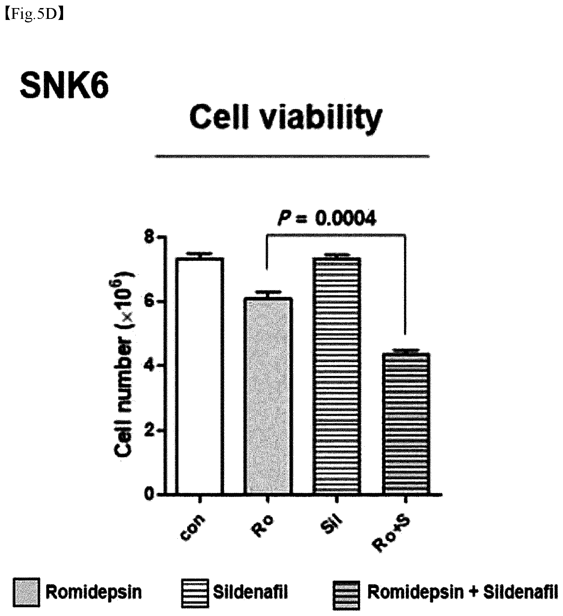

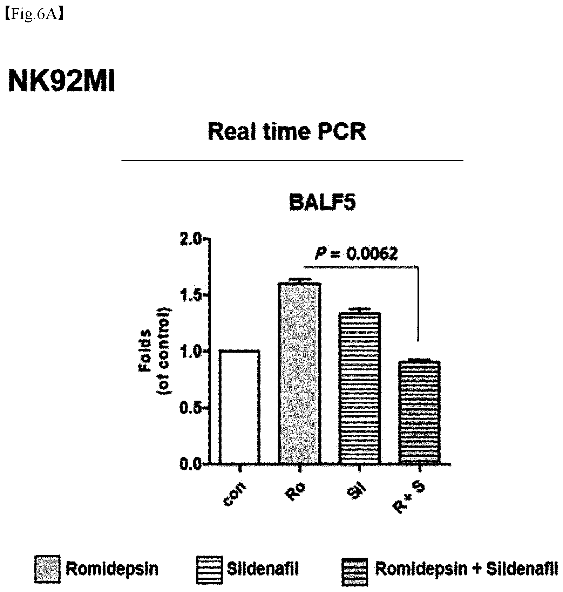

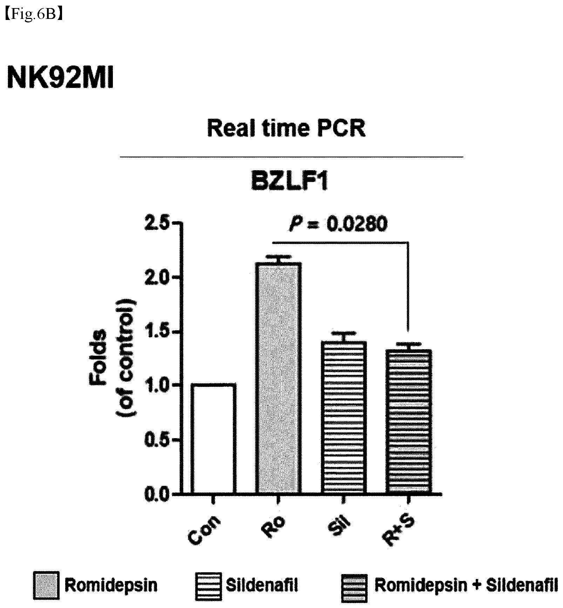

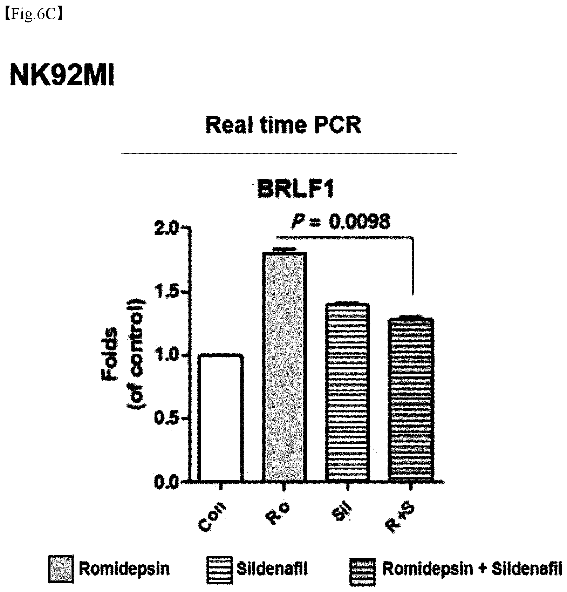

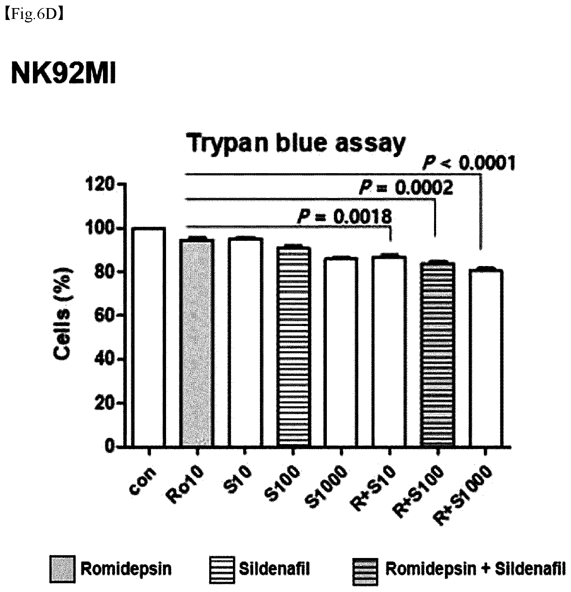

The inhibition of EBV reactivation by sildenafil (100 nM, 72 hours), which was shown by treatment with 10 nM of romidepsin in SNK6 and NK92MI cell lines for 72 hours, was measured through EBV copy number, BALF5, BZLF1, and BRLF1 expression. In addition, cell growth was measured by counting live cells through trypan blue staining. BALF5 is a gene with the function of generating viral DNA produced through EBV reactivation. DNA was extracted from the medium using QIAamp DNA mini kit (Qiagen) and real-time quantitative PCR was performed to measure mRNA of BALF5. Table 2 shows the BALF5 primer sequences used in real-time PCR. The results of measuring the number of EBV copies in the SNK6 cells identified as described above are shown in FIG. 5A, the expression of BZLF1 mRNA is shown in FIG. 5B, the expression of BRLF1 mRNA is shown in FIG. 5C, and the survival rate of tumor cells is shown in FIG. 5D. In addition, the expression of BRLF5 mRNA in NK92MI cells is shown in FIG. 6A, the expression of BZLF1 mRNA is shown in FIG. 6B, the expression of BRLF1 mRNA is shown in FIG. 6C, and the survival rate of tumor cells is shown in FIG. 6D.

**[Table 2]**

| **Primer names** | **Primer Sequences (5' -> 3')** | **SEQ ID NOS.** |
|---|---|---|
| BALF5-Forward primer | CGT CTC ATT CCC AAG TGT TTC | 7 |
| BALF5-Reverse primer | GCC CTT TCC ATC CTC GTC | 8 |

As shown in FIGS. 5A to 5D, when comparing the group administered with romidepsin alone and the group administered with romidepsin and sildenafil in the SNK6 cell line, it was identified that the expressions of EBV copy number, BZLF1, and BRLF1 were decreased in the group administered with romidepsin and sildenafil, identifying that the reactivation of EBV was inhibited. In addition, it was identified that the viability of the SNK6 cell line, which is an NK/T-cell lymphoma cell line, was decreased in the group administered with romidepsin and sildenafil.

In addition, as shown in FIGS. 6A to 6D, when comparing the group administered with romidepsin alone and the group administered with romidepsin and sildenafil in the NK92MI cell line, it was identified that the expressions of BALF5, BZLF1, and BRLF1 were decreased in the group administered with romidepsin and sildenafil. In addition, it was identified that the reactivation of EBV was inhibited, and that the viability of the NK92MI cell line, which is an NK/T-cell lymphoma cell line, was decreased in the group administered with romidepsin and sildenafil, as a result of trypan blue staining.

From the above results, it was identified that sildenafil effectively inhibited EBV, an oncogenic virus.

### Example 5. Identification of EBV Inhibitory Effect by PDE5 Inhibitor other than Sildenafil

In addition, the EBV inhibitory effect of a PDE5 inhibitor other than sildenafil was identified.

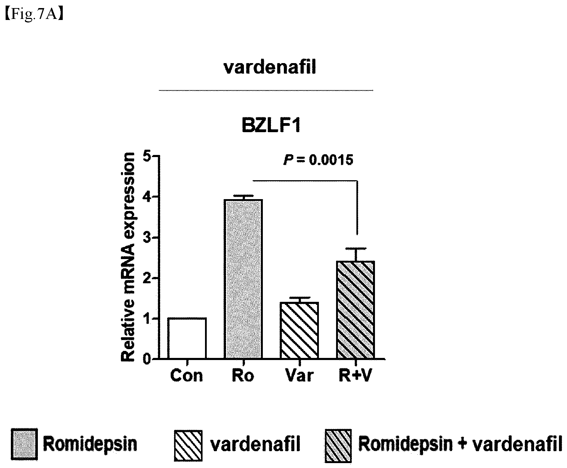

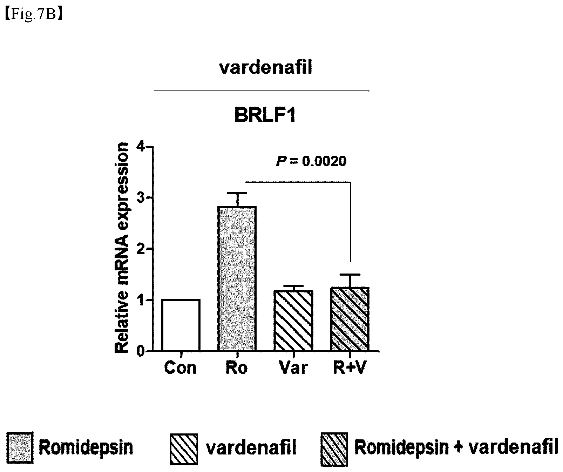

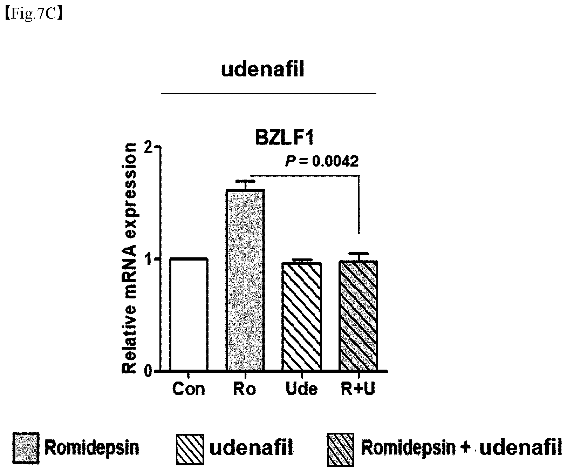

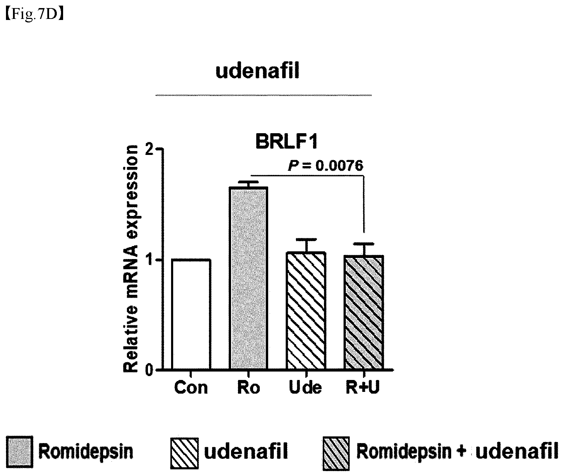

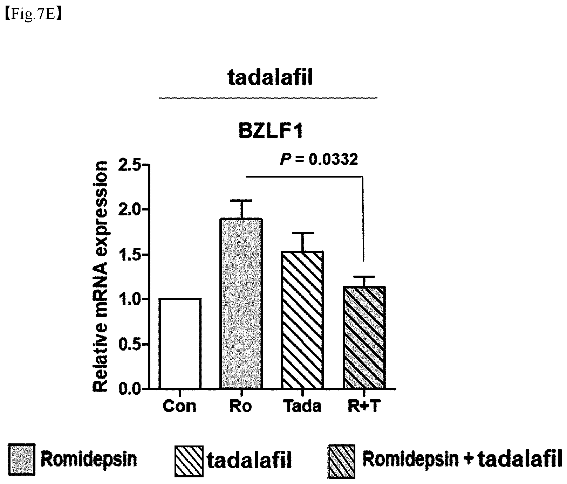

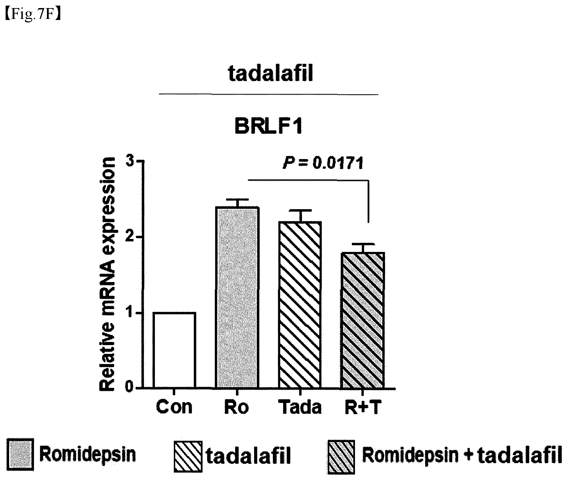

BZLF1 and BRLF1 mRNA expressions were identified and measured by qPCR in the same manner as in Example 3.1 that EBV reactivation, which was shown by treatment of 10 nM of romidepsin in the SNK6 cell line, was inhibited by the PDE5 inhibitors of vardenafil, udenafil or tadalafil. In this connection, vardenafil was treated with 100 nM for 72 hours, udenafil was treated with 100 nM for 48 hours, and tadalafil was treated with 1000 nM for 24 hours. The expression of BZLF1 mRNA in the vardenafil-administered group is shown in FIG. 7A, and the expression of BRLF1 mRNA is shown in FIG. 7B. In addition, the expression of BZLF1 mRNA in the udenafil-administered experimental group is shown in FIG. 7C, and the expression of BRLF1 mRNA is shown in FIG. 7D. In addition, the expression of BZLF1 mRNA in the tadalafil-administered experimental group is shown in FIG. 7E, and the expression of BRLF1 mRNA is shown in FIG. 7F.

As a result, as in the experimental group simultaneously treated with romidepsin and sildenafil in the above example, it was identified that the experimental group treated with vardenafil, udenafil or tadalafil and romidepsin also had decreased BZLF1 and BRLF1 mRNA expressions, identifying that the reactivation of EBV was inhibited.

To sum up, when the NK/T-cell lymphoma cell line and the NK-cell leukemia cell line were simultaneously treated with an HDAC inhibitor and a phosphodiesterase type 5 inhibitor, the reactivation of the EBV induced by the HDAC inhibitor was inhibited, thus identifying that the side effects of the HDAC inhibitor may be decreased, and that the anticancer effect may be significantly increased.

Hereinafter, the formulation examples of a pharmaceutical composition for preventing or treating NK/T-cell lymphoma or NK-cell leukemia including a phosphodiesterase type 5 inhibitor of an embodiment of the present invention will be described. The formulation examples are not intended to limit the present invention, but are merely intended to describe the present invention in detail.

### Formulation Example 1. Preparation of Powders

10 mg of phosphodiesterase type 5 inhibitor of an embodiment of the present invention
1 g of lactose

The above ingredients were mixed and filled in an airtight bag to prepare powders.

### Formulation Example 2. Preparation of Tablets

10 mg of phosphodiesterase type 5 inhibitor of an embodiment of the present invention
100 mg of corn starch
100 mg of lactose
2 mg of magnesium stearate

After mixing the above ingredients, tablets were prepared by tableting according to a conventional preparation method of tablets.

### Formulation Example 3. Preparation of Capsules

10 mg of phosphodiesterase type 5 inhibitor of an embodiment of the present invention
100 mg of corn starch
100 mg of lactose
2 mg of magnesium stearate

According to a conventional preparation method of capsules, the above ingredients were mixed and filled in a gelatin capsule to prepare capsules.

### Formulation Example 4. Preparation of Pills

10 mg of phosphodiesterase type 5 inhibitor of an embodiment of the present invention
1.5 g of lactose
1 g of glycerin
0.5 g of xylitol

After mixing the above ingredients, pills were prepared so as to be 4 g per pill according to a conventional method.

### Formulation Example 5. Preparation of Granules

10 mg of phosphodiesterase type 5 inhibitor of an embodiment of the present invention
50 mg of soybean extract
200 mg of glucose
600 mg of starch

After mixing the above ingredients, 100 mg of 30% ethanol was added and dried at 60°C to form granules, and then filled in a bag.

Hereinbefore, the specific portions of the contents of the present invention have been described in detail. Therefore, it is apparent to a person having ordinary skill in the pertinent art that such specific technology is merely a preferable embodiment, and the scope of the present invention is not limited thereto. Accordingly, the substantial scope of the present invention is defined by the appended claims and their equivalents.

## Claims

1. A pharmaceutical composition for use in preventing or treating natural killer (NK)/T-cell lymphoma or NK-cell leukemia, wherein the pharmaceutical composition comprises a phosphodiesterase type 5 inhibitor, and is for inhibiting reactivation of Epstein-Barr virus (EBV) by a histone deacetylase (HDAC) inhibitor.

2. The pharmaceutical composition of claim 1, wherein the NK/T-cell lymphoma or NK-cell leukemia is EBV-positive NK/T-cell lymphoma or NK-cell leukemia.

3. The pharmaceutical composition of claim 1, wherein the NK/T-cell lymphoma or NK-cell leukemia is EBV-positive NK/T-cell lymphoma or NK-cell leukemia in which the EBV is reactivated by the HDAC inhibitor.

4. The pharmaceutical composition of claim 1, wherein the phosphodiesterase type 5 inhibitor is one or more selected from the group consisting of sildenafil, mirodenafil, vardenafil, tadalafil, udenafil, dasantafil, and avanafil.

5. The pharmaceutical composition of claim 1, wherein the HDAC inhibitor is one or more selected from the group consisting of romidepsin, vorinostat, panobinostat, belinostat, entinostat, mocetinostat, givinostat, pracinostat, quisinostat, abexinostat, chr-3996, and AR-42.

6. A method for treating natural killer (NK)/T-cell lymphoma or NK-cell leukemia, wherein the method comprises administering a phosphodiesterase type 5 inhibitor to a subject, and is for inhibiting reactivation of Epstein-Barr virus by a histone deacetylase inhibitor.

7. A pharmaceutical composition for use in preventing or treating natural killer (NK)/T-cell lymphoma or NK-cell leukemia, wherein the pharmaceutical composition comprises a phosphodiesterase type 5 inhibitor and a histone deacetylase (HDAC) inhibitor.

8. The pharmaceutical composition of claim 7, wherein the NK/T-cell lymphoma or NK-cell leukemia is Epstein-Barr virus-positive.

9. The pharmaceutical composition of claim 7, wherein the NK/T-cell lymphoma or NK-cell leukemia is Epstein-Barr virus (EBV)-positive NK/T-cell lymphoma or NK-cell leukemia in which the EBV is reactivated by the HDAC inhibitor.

10. The pharmaceutical composition of claim 7, wherein the phosphodiesterase type 5 inhibitor is one or more selected from the group consisting of sildenafil, mirodenafil, vardenafil, tadalafil, udenafil, dasantafil, and avanafil.

11. The pharmaceutical composition of claim 7, wherein the HDAC inhibitor is one or more selected from the group consisting of romidepsin, vorinostat, panobinostat, belinostat, entinostat, mocetinostat, givinostat, pracinostat, quisinostat, abexinostat, chr-3996, and AR-42.

12. A method for treating natural killer (NK)/T-cell lymphoma or NK-cell leukemia, wherein the method comprises administering a phosphodiesterase type 5 inhibitor and a histone deacetylase inhibitor to a subject.

13. An anticancer therapy adjuvant composition for use in preventing or treating natural killer (NK)-T cell lymphoma or NK-cell leukemia, wherein the anticancer therapy adjuvant composition comprises a phosphodiesterase type 5 inhibitor, and the NK/T-cell lymphoma or NK-cell leukemia is Epstein-Barr virus-positive.

14. A health functional food for use in preventing or alleviating natural killer (NK)/T-cell lymphoma or NK-cell leukemia, wherein the health functional food comprises a phosphodiesterase type 5 inhibitor, and the NK/T-cell lymphoma or NK-cell leukemia is Epstein-Barr virus-positive.

15. A health functional food for use in preventing or alleviating natural killer (NK)/T-cell lymphoma or NK-cell leukemia, wherein the health functional food comprises a phosphodiesterase type 5 inhibitor and a histone deacetylase inhibitor, and the NK/T-cell lymphoma or NK-cell leukemia is Epstein-Barr virus-positive.
